# EUROPEAN PATENT SPECIFICATION

(11) **EP 2 124 925 B1**
(45) Date of publication and mention of the grant of the patent: **27.06.2018**
(21) Application number: 07847224.8
(22) Date of filing: 20.11.2007
(51) Int. Cl.: A61K 31/366, A61K 31/205, A61K 31/22, A61K 31/405, A61P 3/10, A61K 31/40

(54) **COMPOSITION USEFUL FOR THE TREATMENT OF TYPE 2 DIABETES**
ZUSAMMENSETZUNG ZUR BEHANDLUNG VON DIABETES TYP 2
COMPOSITION À UTILISER POUR LE TRAITEMENT DU DIABÈTE DE TYPE 2

(30) Priority: 27.02.2007 EP 07103137
(43) Date of publication of application: 02.12.2009
(73) Proprietor: Alfasigma S.p.A., Milano (IT)
(72) Inventor: GAETANI, Franco, 00040 Ariccia (RM) (IT); VIRMANI, Ashraf, 00040 Ariccia (RM) (IT)
(74) Representative: Spadaro, Marco
(86) International application number: PCT/EP2007/062545
(87) International publication number: WO 2008/104239

(56) References cited:
- EP-A- 1 623 708
- EP-A1- 0 591 857
- WO-A-00/74675
- US-B1- 6 245 800
- SOLFRIZZI ET AL: "Efficacy and tolerability of combined treatment with l-carnitine and simvastatin in lowering lipoprotein(a) serum levels in patients with type 2 diabetes mellitus" ATHEROSCLEROSIS, AMSTERDAM, NL, vol. 188, no. 2, October 2006 (2006-10), pages 455-461, XP005630950 ISSN: 0021-9150 cited in the application
- F. BRESCIA, E. BALESTRA, M. G. IASELLA, A. B. DAMATO: "Effects of Combined Treatment with Simvastatin and L-Carnitine on Triglyceride Levels in Diabetic Patients with Hyperlipidaemia" CLINICAL DRUG INVESTIGATION, vol. 22, no. suppl1, 2002, pages 23-28, XP009084896
- S. M. HAFFNER: "Diabetes, Hyperlipidemia and Coronary Artery Disease" AMERICAN JOURNAL OF CARDIOLOGY, vol. 83, 1999, pages 17F-21F, XP002437295
- D. W. SECCOMBE, L. JAMES, P. HAHN, E. JONES: "L-Carnitine Treatment in the Hyperlipidemic Rabbit" METABOLISM, vol. 36, no. 12, 1987, pages 1192-1196, XP002437296
- ROSSMANITH R ET AL: "Comparison of the effects of L-carnitine and acetyl-L-carnitine on adipocyte glucose metabolism in spontaneously obese rats", INTERNATIONAL JOURNAL OF OBESITY, NEWMAN PUBLISHING, LONDON, GB, vol. 18, no. SUPPL. 2, 20 August 1994 (1994-08-20), page 64, XP008097783, ISSN: 0307-0565
- COSTA A ET AL: "Effects of atorvastatin on glucose homeostasis, postprandial triglyceride response and C-reactive protein in subjects with impaired fasting glucose", DIABETIC MEDICINE, JOHN WILEY & SONS, LTD, GB, vol. 20, no. 9, 1 January 2003 (2003-01-01), pages 743-745, XP008082705, ISSN: 0742-3071, DOI: 10.1046/J.1464-5491.2003.00993.X

## Description

### Field of the invention

The present invention relates to the use of L-carnitine or an alkanoyl L-carnitine in combination with a statin for the treatment of type 2 diabetes.

### Background of the invention

Diabetes is a widespread disease throughout the world and is associated with major clinical complications involving the microvascular district, such as diabetic retinopathy, diabetic neuropathy and nephropathy, and the macrovascular district, such as atherosclerosis, peripheral vasculopathies, myocardial infarct and stroke.

Insulin resistance, which characterises type 2 diabetes and its micro- and macrovascular complications is also involved in syndrome X, polycystic ovary syndrome, obesity, hypertension, hyperlipidaemias and hypercholesterolaemias (J. Am. Osteopath. Assoc., 2000 Oct.; 100(10):621-34*;* Jama, 2002 Nov., 27;288 (20):2579-88).

It is known that hyperlipidaemias, hypercholesterolaemias and hypertension play a decisive role in the onset of coronary heart disease (CHD).

It is well known that an increase in glycosylation of proteins is involved in all the above-mentioned complications of diabetes (Diabetologia 2001 Feb; 44(2):129-46).

Said complications constitute a serious threat to the life and well-being of the individual.

Various clinical forms of diabetic disease are known, the most common being type 2 and type 1 diabetes. Type 2 diabetes is characterised by reduced sensitivity to the action of insulin (insulin resistance) and gives rise to an increase in insulin levels in the body in an attempt to compensate for this deficiency and to a consequent increase in glucose levels.

People with blood glucose levels that are higher than normal but not yet in the diabetic range have "pre-diabetes".

Insulin resistance is a silent condition that increases the chances of developing diabetes. In insulin resistance condition the muscle, fat, and liver cells do not use insulin properly. The pancreas tries to keep up with the demand for insulin by producing more. Excess weight also contributes to insulin resistance because too much fat interferes with muscles' ability to use insulin. Lack of exercise further reduces muscles' ability to use insulin.

Insulin Resistance and obesity-linked to pre-diabetes can be an increased risk factor for hypertension, or high blood pressure which is one of the most important risk factors for cardiovascular disease, which can lead to a heart attack or stroke. If left untreated, hypertension can also lead to a wide variety of other life-threatening conditions, such as kidney damage and congestive heart failure.

Diabetes or pre-diabetes can be detected with one of the following tests:
- Fasting Glucose Test, which measures blood glucose after not eating overnight. This test is most reliable when done in the morning. Fasting glucose levels of 100 to 125 mg/dL are above normal but not high enough to be called diabetes. This condition is called pre-diabetes or impaired fasting glucose (IFG), and it suggests that patient has probably had insulin resistance for some time. IFG is considered a pre-diabetic state, meaning that the patient is are more likely to develop diabetes but does not yet have it.
- Glucose Tolerance Test, which measures blood glucose after an overnight fast and 2 hours after patient drinks a sweet liquid provided by the doctor or laboratory. If patient blood glucose falls between 140 and 199 mg/dL, 2 hours after drinking the liquid, patient glucose tolerance is above normal but not high enough for diabetes. This condition, also a form of pre-diabetes, is called impaired glucose tolerance (IGT) and, like IFG, it points toward a history of insulin resistance and a risk for developing diabetes.

Insulin resistance can be assessed with measurement of fasting insulin.

Numerous reports have confirmed the involvement of insulin resistance in many disease conditions in addition to type 2 diabetes itself, such as dyslipidaemia, obesity, arterial hypertension and certain macrovascular and microvascular manifestations characteristic of diabetic disease itself. The combination of insulin resistance and obesity, hypertension and dyslipidaemia is known as Syndrome X.
Drugs used for many years such as the biguanides and sulphonylurea drugs are available on the market for the treatment of type 2 diabetes. Many of these, such as, for example, methformin, present gastrointestinal disorders, danger of acidosis in conditions of renal, cardiac, hepatic, pulmonary insufficiency, etc., as side effects. The sulphonylureas have episodes of hypoglycaemia as their possible side effects. Drugs recently introduced onto the market are the thiazolidonides, whose side effects such as liver toxicity, increased LDL cholesterol, weight gain and oedema have given cause for concern.
Hyperlipidaemia is a serious aspect of diabetic disease, constituting, together with the hypertension which is often present, a risk factor for atherosclerosis and for cardiovascular disease which is the primary cause of death in diabetes.

Cardiovascular disease is recognised as the primary cause of death in the industrialised countries with a high standard of living.
The social cost is enormous, both in terms of disability and invalidity of subjects suffering from it, and in terms of the actual cost of health facilities and insurance.

Dyslipidaemia is often associated, also as a consequence, with diabetes.

In WO 99/01126 is described a combination of statin and alkanoyl L-carnitines useful for treating diseases due to an altered lipid metabolism.

In WO0074675 is described the use of carnitines for reducing the toxicity due to the administration of statins.

In Clin Ter. 1992 Jan; 140(1 Pt 2):17-22 is described the hypotriglyceridemic action of L-carnitine in combination with simvastatin, in patient with renal failure.

In Atherosclerosis 188, 2006, 455-461 is described the efficacy of L-carnitine in combination with simvastatin in lowering Lipoprotein(a), in patient with type 2 diabetes.

In Minerva Medica, Vol.80, N° 3 is described the use of L-carnitine for the treatment of hypertension in patient with type 2 diabetes.

In Muscle & Nerves 34: August 2006, 153-162, is reported that the use of statins in patients with fatty acid oxidation defects and mitochondrial disorders, including carnitine abnormalities, causes an higher prevalence of metabolic muscle diseases than expected in general population.
In these documents there is never any mention of insulin resistance or reduction of protein glycosylation.
Ever increasing attention is being devoted to the so-called risk factors recognised as underlying these diseases, and there is still a perceived need for a medicine capable of acting on the various sources of this pathological picture, without, at the same time, being associated with severe side effects, which, as in the case of certain antidiabetic drugs, may even make it necessary to discontinue the therapy.

### Description of the invention

It has now surprisingly been found that a certain combination of substances, known for their specific pharmacological actions, is particularly indicated for the treatment of type 2 diabetes.

The combination according to the invention comprises L-carnitine and/or one or more alkanoyl L-carnitines, or one of their pharmaceutically acceptable salts, and a statin.

What is meant by pharmaceutically acceptable salt of L-carnitine is any salt of the latter with an acid that does not give rise to toxic or side effects. These acids are well known to pharmacologists and to experts in pharmacy. Non-limiting examples of such salts are: chloride, bromide, orotate, aspartate, acid aspartate, acid citrate, magnesium citrate, phosphate, acid phosphate, fumarate and acid fumarate, magnesium fumarate, lactate, maleate and acid maleate, oxalate, acid oxalate, pamoate, acid pamoate, sulphate, acid sulphate, glucose phosphate, tartrate and acid tartrate, glycerophosphate, mucate, magnesium tartrate, 2-amino-ethanesulphonate, magnesium 2-amino-ethanesulphonate, methanesulphonate, choline tartrate, trichloroacetate, and trifluoroacetate.

What is meant by pharmaceutically acceptable salt of L-carnitine is also a salt approved by the FDA and listed in the publication Int. J. of Pharm. 33 (1986), 201-217. The combination according to the present invention exerts a surprising synergistic effect on insulin resistance and reduction of protein glycosylation, which is not predictable on the basis of our knowledge of the individual components thereof.

The advantage of having such a combination is therefore evident to experts in the sector. It is possible, in fact, to treat insulin resistance, to reduce protein glycosylation.

Therefore, one object of the present invention is the use of L-carnitine and/or of one or more alkanoyl L-carnitines selected from the group consisting of acetyl, propionyl, valeryl, isovaleryl, butyryl and isobutyryl L-carnitine, or one of their pharmaceutically acceptable salts, in combination with a statin selected from the group consisting of simvastatin, lovastatin, fluvastatin, pravastatin, atorvastatin, cerivastatin, rovastatin and rosuvastatin, the one preferred is simvastatin, for preparing a medicament for the treatment of type 2 diabetes, for reducing protein glycosylation.

The combination according to the invention can also comprise other useful elements, without this substantially impairing the activity.

The combination according to the present invention can also be formulated as a food supplement, which constitutes a further object of the invention. Other objects of the present invention are various uses of the above-mentioned combination as a medicine, in particular for the preparation of a medicine for the treatment of insulin resistance and type 2 diabetes

In particular, the present invention provides for the use of the above-mentioned combination for the preparation of a medicine useful for the treatment of diseases involving insulin resistance such as type 2 diabetes

The medicine according to the invention can be used to treat the individual disease states or to exert a preventive or protective action against them, or to treat a complex pathological picture that includes one or more of the therapeutic aspects seen above. For example, a medicine with a combined action for the reduction of protein glycosylation, the treatment of type 2 diabetes and insulin resistance and an antilipaemic and protective action on the cardiovascular system, particularly in certain severe forms of type 2 diabetes associated with obesity.

### Detailed description of the invention

The combination according to the present invention comprises as active ingredients which are known in the medical sector and already used in clinical practice. Therefore, they are very easy to procure, inasmuch as they are products which have been on the market for some time and are of a grade suitable for human or animal administration.

The statins are a known class of drugs used for lowering cholesterol levels. Statins are available on the market or can be prepared according to known methods described in the literature. L-carnitine and its alkanoyl derivatives are known compounds, the preparation process for which is described in US 4,254,053.

Several previous therapeutic uses of carnitines in treating the diabetes are already known.

For example, WO 98/01128 discloses the use of the acetil L-carnitine, isovaleril L-carnitine, propionil L-carnitine to increase the levels of IGF-1.

The diabetes is also included in the long list of curable pathologies stated in WO 98/01128.

WO 98/41113 describes a therapeutic nutritive composition for patients with diabetes mellitus consisting of gamma linoleic acid, acetil L-carnitine, mineral salts and vitamins.

US 4.362.719 describes the use of the L-carnitine and the acil L-carnitine in treating the juvenile onset diabetes mellitus.

US 5.430.065 describes the use of the L-carnitine and the acil L-carnitine in the long-term treatment of those patients with noninsulin-dependent diabetes.

In Journal of Cellular Physiology 203; 2005; 439-446 is reported that the addition of acetyl L-carnitine to the culture medium dramatically affected the ability of myocites to respond to insulin treatment.

None of the publication above mentioned describe that L-carnitine and/or of one or more alkanoyl L-carnitines in combination with a statin would have been useful for preparing a medicament for the treatment of type 2 diabetes and diseases related protein glycosylation due to type 2 diabetes. According to the present invention, it is also possible to combine a number of statins with one or more carnitines, depending on their pharmacological characteristics and on the basis of the common knowledge of experts in the sector.

This implies that, apart from the consideration of the synergistic effect demonstrated here below, the dosages and ratios of the individual components can be determined by the expert in the sector with normal preclinical and clinical trials, or with the usual considerations regarding the formulation of a dietetic product.

The amounts of the individual compounds advised for the preparation of a pharmaceutical composition for human use are the following.

Simvastatin: from 5 mg to 80 mg/day, preferably 15 to 40 mg/day; most preferably 20 mg/day. L-carnitine and/or an alkanoyl L-carnitine: from 0.5 to 5 g/day, preferably 1.5 to 3 g/day; most preferably 2 g/day.

The pharmaceutical composition can have a unitary form, in which the active ingredients are present in a single pharmaceutical form (tablet, sachet, capsule, vial) or the active ingredients can be administered in a coordinated sequential manner. In the latter case, the pharmaceutical composition can be formulated, supplying the components in separate containers, accompanied by instructions for their sequential administration. The compositions covered by the present invention are entirely conventional and are obtained with methods that are common practice in the pharmaceutical industry. According to the administration route opted for, the compositions will be in solid or liquid form, suitable for oral, parenteral or intravenous administration. The compositions according to the present invention contain, along with the active ingredient, at least one pharmaceutically acceptable vehicle or excipient. Particularly useful may be formulation adjuvants such as, for example, solubilising agents, dispersing agents, suspension agents and emulsifying agents. A general reference work is Remington's Pharmaceutical Sciences Handbook, latest edition.

As mentioned above, insulin resistance and diabetes can be an increased risk factor for hypertension, or high blood pressure which is one of the most important risk factors for cardiovascular disease, which can lead to a heart attack or stroke. If left untreated, hypertension can also lead to a wide variety of other life-threatening conditions, such as kidney damage and congestive heart failure. The composition of the invention can be administered with known drugs useful for treating hypertension, or with further antidiabetic drugs, according to the physician prescription and experience.

The following examples further illustrate the invention.

### EXAMPLE 1

### Antidiabetic and serum lipid-lowerinq activity in db/db mice

Mutations in laboratory animals have made it possible to develop models that present non-insulin-dependent diabetes associated with obesity, hyperlipidaemia and insulin resistance and that enable us to test the efficacy of new antidiabetic compounds (Reed and Scribner, Diabetes, obesity and metabolism 1: 75 - 86, 1999).

A much used genetically diabetic mouse model is the C57BL/KsJ db/db mouse.

The genetic basis of this model is a defect in the leptin receptor gene which gives rise to leptin resistance and leads to hyperphagia, obesity, hyperinsulinaemia and insulin resistance, with subsequent symptoms of insufficient insulin secretion and hyperglycaemia (Kodama et al., Diabetologia 37: 739 - 744, 1994; Chen et al, Cell 84: 491 - 495, 1996).

Since hyperglycaemia is accompanied by obesity and insulin resistance, the db/db mouse has characteristics that are close to those of type 2 diabetes in man and is useful for assaying insulin-sensitising compounds.

The C57BL/KsJ db/db mice used in the experiments were supplied by Jackson Lab (via Ch. River). After 10 days of acclimatisation in standard conditions (22±2[deg.]C; 55±15% humidity; 15-20 air changes/hour; 12 hour light-darkness cycle with light from 7 a.m. to 7 p.m.) on a standard 4 RF21 diet (Mucedola), blood samples were taken in post- absorption conditions (fasting from 8.30 a.m. to 4.30 p.m.) from the caudal vein with the aid of a Jelco 22G catheter (Johnson and Johnson). Glucose, insulin, triglyceride, cholesterol, free fatty acid and urea levels were checked in the plasma to ensure well-matched distribution of the mice in the treatment groups.

At the start of treatment, the body weight of the animals was checked and monitoring of the animals' consumption of water and feed was scheduled. The mice were divided into groups and treated orally twice daily with:
Simvastatin 100 mg/kg;
L-carnitine inner salt 400 mg/kg;
acetyl L-carnitine HCI 592 mg/kg (equimolar amount respect to L-carnitine);
propionyl L-carnitine HCI 627 mg/kg (equimolar amount respect to L-carnitine);
L-carnitine inner salt 400 mg/kg + Simvastatin 100 mg/kg;
acetyl L-carnitine HCI 592 mg/kg + Simvastatin 100 mg/kg;
propionyl L-carnitine HCI 627 mg/kg + Simvastatin 100 mg/kg.

In the course of the experiment, serum glucose levels, glucose tolerance (OGTT), a number of lipid status variables and weight gain were monitored. The combination according to the invention was capable of lowering serum glucose levels in the feeding condition (Table 1); in the post- absorption condition (Table 2); and in the fasting condition (Table 3); and capable of improving glucose tolerance (Table 4), and of reducing the levels of fructosamine, an indicator of protein glycosylation (Table 5) which, as mentioned above, plays an important role in the development of the micro- and macrovascular complications of diabetes.

The combination according to the invention also shows good ability to reduce serum triglyceride levels (Table 6) and to increase HDL- cholesterol levels (Table 7).

An increase in HDL-cholesterol values constitutes an indicator of a reduced risk of atherosclerosis and of cardiovascular complications such as atherosclerosis and infarct.

**TABLE 1**

| Blood glucose levels of db/db mice, treated orally twice daily for 12 days with the compounds and at the doses indicated in the table. Sample in feeding condition, approximately 15 hours after the last treatment. | | | |
|---|---|---|---|
| **Compound** | **Glucose mg/dl Mean values** | **± S.D.** | ***P* (Student's t-test)** |
| Control | 483.1 | 14.8 | |
| Simvastatin | 465.0 | 19.4 | NS |
| L-carnitine | 470.0 | 25.9 | NS |
| Propionyl L-carnitine | 475.5 | 28.8 | NS |
| Acetyl L-carnitine | 468.0 | 25.7 | NS |
| L-carnitine + Simvastatin | 294.1 | 33.1 | P<0.001 vs control |
| Propionyl L-carnitine + Simvastatin | 303.5 | 21.1 | P<0.001 vs control |
| Acetyl L-carnitine + Simvastatin | 304.6 | 15.6 | P<0.001 vs control |

| | | | |
|---|---|---|---|
| Number of animals per group: 6. | | | |

**TABLE 2**

| Blood glucose levels of db/db mice, treated orally twice daily for 12 days with the compounds and at the doses indicated in the table. Sample in post-absorption condition (fasting from 9 a.m. to 5 p.m.) and 8 hours after the last treatment. | | | |
|---|---|---|---|
| **Compound** | **Glucose mg/dl Mean values** | **± S.D.** | ***P* (Student's t-test)** |
| Control | 410.8 | 10.4 | |
| Simvastatin | 418.1 | 20.6 | NS |
| L-carnitine | 416.5 | 22.6 | NS |
| Propionyl L-carnitine | 411 | 7.4 | NS |
| Acetyl L-carnitine | 416.1 | 25.5 | NS |
| L-carnitine + Simvastatin | 220.5 | 20.8 | P<0.001 vs control |
| Propionyl L-carnitine + Simvastatin | 218.0 | 14.9 | P<0.001 vs control |
| Acetyl L-carnitine + Simvastatin | 215.5 | 16.1 | P<0.001 vs control |

| | | | |
|---|---|---|---|
| Number of animals per group: 6. | | | |

**TABLE 3**

| Blood glucose levels of db/db mice, treated orally twice daily for 18 days with the compounds and at the doses indicated in the table. Sample in mice fasted for 18 hours and 5 hours after the last treatment. | | | |
|---|---|---|---|
| **Compound** | **Glucose mg/dl Mean values** | **± S.D.** | ***P* (Student's t-test)** |
| Control | 342.5 | 20.1 | |
| Simvastatin | 328.3 | 21.76 | NS |
| L-carnitine | 324.8 | 18.6 | NS |
| Propionyl L-carnitine | 328.6 | 16.3 | NS |
| Acetyl L-carnitine | 332.0 | 15.5 | NS |
| L-carnitine + Simvastatin | 153.83 | 7.63 | P<0.001 vs control |
| Propionyl L-carnitine + Simvastatin | 143.8 | 6.5 | P<0.001 vs control |
| Acetyl L-carnitine + Simvastatin | 147.8 | 4.3 | P<0.001 vs control |

| | | | |
|---|---|---|---|
| Number of animals per group: 6. | | | |

**TABLE 4**

| Area under the curve (AUC) of the OGTT in the blood of db/db mice, treated orally twice daily for 18 days with the compounds and at the doses indicated in the table. | | | |
|---|---|---|---|
| OGTT test (glucose 3 g/kg) in mice fasted for 18 hours and 5 hours after the last treatment. | | | |
| **Compound** | **AUC Glucose u.a. Mean values** | ± □**S.D.** | ***P* Student's t-test** |
| Control | 52447.7 | 1950.6 | |
| Simvastatin | 50973.3 | 2950.3 | NS |
| L-carnitine | 50187.8 | 2557.7 | NS |
| Propionyl L-carnitine | 49005.5 | 3840.8 | NS |
| Acetyl L-carnitine | 49332.3 | 366.3 | NS |
| L-carnitine + Simvastatin | 36149.5 | 2367.1 | P<0.001 vs control |
| Propionyl L-carnitine + Simvastatin | 34695 | 2617.7 | P<0.001 vs control |
| Acetyl L-carnitine + Simvastatin | 35786.5 | 1795.6 | P<0.001 vs control |

| | | | |
|---|---|---|---|
| Number of animals: 6. | | | |

**TABLE 5**

| Plasma fructosamine levels of db/db mice, treated orally twice daily for 25 days with the compounds and at the doses indicated in the table. | | | |
|---|---|---|---|
| Sample in post-absorption condition (fasting from 9 a.m. to 4.30 p.m.) and 7.5 hours after the last treatment. | | | |
| **Compound** | **Fructosamine mM Mean values** | ± □**S.D.** | ***P* Student's t-test** |
| Control | 0.82 | 0.03 | |
| Simvastatin | 0.76 | 0.08 | NS |
| L-carnitine | 0.81 | 0.06 | NS |
| Propionyl L-carnitine | 0.81 | 0.04 | NS |
| Acetyl L-carnitine | 0.85 | 0.03 | NS |
| L-carnitine + Simvastatin | 0.49 | 0.07 | P<0.001 vs control |
| Propionyl L-carnitine + Simvastatin | 0.54 | 0.05 | P<0.001 vs control |
| Acetyl L-carnitine + Simvastatin | 0.56 | 0.04 | P<0.001 vs control |

| | | | |
|---|---|---|---|
| Number of animals per group: 6. | | | |

**TABLE 6**

| Plasma triglyceride levels of db/db mice, treated orally twice daily for 25 days with the compounds and at the doses indicated in the table. | | | |
|---|---|---|---|
| Sample in post-absorption condition (fasting from 9 a.m. to 4.30 p.m.) and 7.5 hours after the last treatment. | | | |
| **Compound** | **Triglycerides mg/dl Mean values** | ±□ **S.D.** | ***P* Student's t-test** |
| Control | 90.6 | 4.1 | |
| Simvastatin | 83.6 | 5.8 | P<0.05 |
| L-carnitine | 85.7 | 3.8 | NS |
| Propionyl L-carnitine | 86.2 | 4.2 | NS |
| Acetyl L-carnitine | 85.6 | 4.4 | NS |
| L-carnitine + Simvastatin | 64.4 | 4.5 | P<0.001 vs control |
| Propionyl L-carnitine + Simvastatin | 55.8 | 3.9 | P<0.001 vs control |
| Acetyl L-carnitine + Simvastatin | 49.4 | 2.3 | P<0.001 vs control |

| | | | |
|---|---|---|---|
| Number of animals per group: 6. | | | |

**TABLE 7**

| Plasma HDL-cholesterol levels of db/db mice, treated orally twice daily for 25 days with the compounds and at the doses indicated in the table. | | | |
|---|---|---|---|
| Sample in post-absorption conditions (fasting from 9 a.m. to 4.30 p.m.) and 7.5 hours after the last treatment. | | | |
| **Compound** | **HDL-cholesterol mg/dl Mean values** | **± S.D.** | ***P* Student's t-test** |
| Control | 80.9 | 3.9 | |
| Simvastatin | 74.5 | 2.4 | P<0.01 |
| L-carnitine | 84.0 | 4.4 | NS |
| Propionyl L-carnitine | 80.9 | 2.3 | NS |
| Acetyl L-carnitine | 84.5 | 3.7 | NS |
| L-carnitine + Simvastatin | 91.2 | 1.8 | P<0.001 vs control |
| Propionyl L-carnitine + Simvastatin | 91.4 | 2.3 | P<0.001 vs control |
| Acetyl L-carnitine + Simvastatin | 91.4 | 2.8 | P<0.001 vs control |

| | | | |
|---|---|---|---|
| Number of animals per group: 6. | | | |

The results reported above clearly demonstrate the unexpected synergism of the combination according to the present invention, respect to the single components.

## Claims

1. L-carnitine and/or an alkanoyl L-carnitine, or a pharmaceutically acceptable salt thereof, in combination with a statin, for use for the treatment of type 2 diabetes.

2. Combination for use according to claim 1 in which the alkanoyl L-carnitine is selected from the group consisting of acetyl, propionyl, valeryl, isovaleryl, butyryl and isobutyryl L-carnitine.

3. Combination for use according to claim 1 in which the pharmaceutically acceptable salt of L-carnitine or the alkanoyl L-carnitine is selected from the group consisting of chloride, bromide, orotate, aspartate, acid aspartate, acid citrate, magnesium citrate, phosphate, acid phosphate, fumarate and acid fumarate, magnesium fumarate, lactate, maleate and acid maleate, oxalate, acid oxalate, pamoate, acid pamoate, sulphate, acid sulphate, glucose phosphate, tartrate and acid tartrate, glycerophosphate, mucate, magnesium tartrate, 2-amino-ethanesulphonate, magnesium 2-amino-ethanesulphonate, methanesulphonate, choline tartrate, trichloroacetate, and trifluoroacetate.

4. Combination for use according to claim 1 in which the statin is selected from the group consisting of simvastatin, lovastatin, fluvastatin, pravastatin, atorvastatin, cerivastatin, rovastatin and rosuvastatin.

5. Combination for use according to claim 4 in which the statin is simvastatin.

6. Combination for use according to claim 1 in which the medicament to be administered comprises a statin in an amount of from 5 mg to 80 mg/day, preferably 15 to 40 mg/day; most preferably 20 mg/day; and the carnitine and/or the derivative thereof in an amount of from 0.5 to 5 g/day, preferably 1.5 to 3 g/day; most preferably 2 g/day.

7. Combination for use according to claim 6 in which the medicament is in solid or liquid form, suitable for oral or parenteral administration in the form of tablet, sachet, capsule or vial.

8. Combination for use according to claim 6 in which the medicament is in a single pharmaceutical form or in separate containers for sequential administration.

## Patentansprüche

1. L-Carnitin und/oder ein Alkanoyl-L-carnitin, oder ein pharmazeutisch annehmbares Salz hiervon, in Kombination mit einem Statin, für die Verwendung zur Behandlung von Typ-2-Diabetes.

2. Kombination zur Verwendung, gemäß Anspruch 1, in welcher das Alkanoyl-L-carnitin ausgewählt ist aus der Gruppe bestehend aus Acetyl-, Propionyl-, Valeryl-, Isovaleryl-, Butyryl- und Isobutyryl-L-carnitin.

3. Kombination zur Verwendung, gemäß Anspruch 1, in welcher das pharmazeutisch annehmbare Salz von L-Carnitin oder das Alkanoyl-L-carnitin ausgewählt ist aus der Gruppe bestehend aus Chlorid, Bromid, Orotat, Aspartat, saurem Aspartat, saurem Citrat, Magnesiumcitrat, Phosphat, saurem Phosphat, Fumarat und saurem Fumarat, Magnesiumfumarat, Laktat, Maleat und saurem Maleat, Oxalat, saurem Oxalat, Pamoat, saurem Pamoat, Sulfat, saurem Sulfat, Glucosephosphat, Tartrat und saurem Tartrat, Glycerophosphat, Mucat, Magnesiumtartrat, 2-Amino-ethansulfonat, Magnesium-2-amino-ethansulfonat, Methansulfonat, Cholintartrat, Trichloracetat und Trifluoracetat.

4. Kombination zur Verwendung, gemäß Anspruch 1, in welcher das Statin ausgewählt ist aus der Gruppe bestehend aus Simvastatin, Lovastatin, Fluvastatin, Pravastatin, Atorvastatin, Cerivastatin, Rovastatin und Rosuvastatin.

5. Kombination zur Verwendung, gemäß Anspruch 4, in welcher das Statin Simvastatin ist.

6. Kombination zur Verwendung, gemäß Anspruch 1, in welcher das zu verabreichende Arzneimittel ein Statin in einer Menge von 5 mg bis 80 mg/Tag, bevorzugt 15 bis 40 mg/Tag; am meisten bevorzugt 20 mg/Tag; und das Carnitin und/oder dessen Derivat in einer Menge von 0,5 bis 5 g/Tag, bevorzugt 1,5 bis 3 g/Tag; am meisten bevorzugt 2 g/Tag umfasst.

7. Kombination zur Verwendung, gemäß Anspruch 6, in welcher das Arzneimittel in fester oder flüssiger Form ist, geeignet zur oralen oder parenteralen Verabreichung in Form von Tablette, Sachet, Kapsel oder Ampulle.

8. Kombination zur Verwendung, gemäß Anspruch 6, in welcher das Arzneimittel in einer pharmazeutischen Einzelform oder in getrennten Behältern für sequentielle Verabreichung vorliegt.

## Revendications

1. L-carnitine et/ou une alcanoyl L-carnitine, ou un sel pharmaceutiquement acceptable de celles-ci, en combinaison avec une statine, pour utilisation dans le traitement du diabète de type 2.

2. Combinaison pour utilisation selon la revendication 1, dans laquelle l'alcanoyl L-carnitine est choisie dans le groupe constitué de l'acétyl, la propionyl, la valéryl, l'isovaléryl, la butyryl et l'isobutyryl L-carnitine.

3. Combinaison pour utilisation selon la revendication 1, dans laquelle le sel pharmaceutiquement acceptable de la L-carnitine ou de l'alcanoyl L-carnitine est choisi dans le groupe constitué du chlorure, du bromure, de l'orotate, de l'aspartate, de l'aspartate acide, du citrate acide, du citrate de magnésium, du phosphate, du phosphate acide, du fumarate et du fumarate acide, du fumarate de magnésium, du lactate, du maléate et du maléate acide, de l'oxalate, de l'oxalate acide, du pamoate, du pamoate acide, du sulfate, du sulfate acide, du phosphate de glucose, du tartrate et du tartrate acide, du glycérophosphate, du mucate, du tartrate de magnésium, du 2-amino-éthanesulfonate, du 2-amino-éthanesulfonate de magnésium, du méthanesulfonate, du tartrate de choline, du trichloroacétate et du trifluoroacétate.

4. Combinaison pour utilisation selon la revendication 1, dans laquelle la statine est choisie dans le groupe constitué de la simvastatine, la lovastatine, la fluvastatine, la pravastatine, l'atorvastatine, la cérivastatine, la rovastatine et la rosuvastatine.

5. Combinaison pour utilisation selon la revendication 4, dans laquelle la statine est la simvastatine.

6. Combinaison pour utilisation selon la revendication 1, dans laquelle le médicament à administrer comprend une statine dans une quantité de 5 mg à 80 mg/jour, de préférence de 15 à 40 mg/jour; plus préférentiellement de 20 mg/jour; et la carnitine et/ou le dérivé de celle-ci dans une quantité de 0,5 à 5 g/jour, de préférence de 1,5 à 3 g/jour; plus préférentiellement de 2 g/jour.

7. Combinaison pour utilisation selon la revendication 6, dans laquelle le médicament est sous une forme solide ou liquide, convenant à une administration orale ou parentérale sous la forme d'un comprimé, d'un sachet, d'une capsule ou d'une ampoule.

8. Combinaison pour utilisation selon la revendication 6, dans laquelle le médicament est sous une forme pharmaceutique unique ou dans des contenants séparés pour une administration séquentielle.
